# EUROPEAN PATENT SPECIFICATION

(11) **EP 1 509 190 B1**
(45) Date of publication and mention of the grant of the patent: **08.03.2006**
(21) Application number: 03730113.2
(22) Date of filing: 22.05.2003
(51) Int. Cl.: A61Q 1/00, A61Q 17/04, A61Q 19/00, A61K 8/36, A61K 8/362, A61K 8/41, A61K 8/44, A61K 8/02

(54) **ODOR CONTROL IN AMINE SALT CONTAINING COSMETIC COMPOSITIONS**
REGULIERUNG DES GERUCHS VON KOSMETISCHEN ZUSAMMENSETZUNGEN, WELCHE AMINSALZE ENTHALTEN
MAITRISE DE L'ODEUR DANS DES COMPOSITIONS COSMETIQUES CONTENANT UN SEL D'AMINE

(30) Priority: 29.05.2002 US 383837 P; 17.03.2003 US 455331 P
(43) Date of publication of application: 02.03.2005
(73) Proprietor: UNILEVER PLC, London EC4P 4BQ (GB); UNILEVER N.V., 3013 AL Rotterdam (NL)
(72) Inventor: FARYNIARZ, Joseph, Unilever Home & Personal Care, Trumbull, CT 06611 (US); ZHANG, Joanna H., Unilever Home & Personal Care, Trumbull, CT 06611 (US)
(74) Representative: Acham, Nicholas Clive
(86) International application number: PCT/EP2003/005478
(87) International publication number: WO 2003/099238

(56) References cited:
- EP-A- 0 396 857
- EP-A- 1 090 630
- EP-A- 1 262 166
- EP-A- 1 295 590
- WO-A-01/17486
- GB-A- 1 310 658
- US-A- 4 419 343
- US-A- 4 469 684
- US-A- 5 258 174
- US-A- 5 352 695

## Description

The invention concerns cosmetic compositions containing ammonium salts, particularly those with organic acids, wherein is achieved control of odor emanating from released free amines.

Amines of a sufficiently low molecular weight to be volatile leave an identifiable odor. As a class of materials, volatile amines smell fish-like. This odor is usually unpleasant.

Volatility and thereby smell is greatly reduced when the amines are quaternized in salt form. Quaternized amines have found particular usefulness in cosmetic compositions. They have been used as cationic counterions to alpha-hydroxycarboxylic acids. Representative disclosures include U.S. Patent 4,105,782, U.S. Patent 4,105,783, U.S. Patent 4,197,316, U.S. Patent 4,234,599 and U.S. Patent 5,091,171 all to Yu and Van Scott.

WO 01/85129 A2 (Cole et al.) disclose the N,N-dimethylethanolammonium (DMAE) salts of dermatologically active acids. The latter are identified as alpha-hydroxy acid, ascorbic acid, lipoic acid and combinations thereof. Besides use of a first neutralizing agent such as DMAE, the disclosure suggests use of a second neutralizing agent which may be an inorganic (e.g. sodium or potassium hydroxide), an amine (e.g. triethanolamine) or an amino acid (e.g. arginine, lysine or tyrosine).

Organic acids other than alpha-hydroxy functionalized ones have been disclosed in the cosmetic literature. For instance, U.S. Patent 5,641,495 (Jokura et al.) discloses in combination a ceramide or pseudoceramide with a dicarboxylic acid and a salt of a dicarboxylic acid. Malonic acid is the lowest molecular weight species of the group. The acids can be neutralized with alkanolamines, basic amino acids and ammonia.

Hitherto the art has not focused upon the latent odor problem. This may have resulted from preoccupation with identifying dermatologically active ingredients, rather than any concern over aesthetics.

A particular advantage of the present invention is that a solution is proposed to the problem caused by release of free amine.

Thus, according to a first aspect there is provided a cosmetic composition which includes:
(i) a malonate salt formed from a first amine which is ammonia or dimethylaminoethanol and malonic acid;
(ii) a second amine different from the first amine and having a molecular weight no lower than 100; and
(iii) from 1% to 99.9% by weight of a cosmetically acceptable carrier.

Now it has been found that formulating a second amine into a cosmetic composition containing a cationic ammonium salt (formed by a first amine reacting with malonic acid) prevents release of the first amine as a smelly volatile substance. Release often happens when the cosmetic composition is rubbed into the skin. Although not wishing to be bound by any theory, it is possible that the high pH of skin releases the first amine from the salt thereby giving rise to the odor.

Sometimes a formulator may desire just the slightest hint of an amine smell as product is rubbed into the skin. By use of the second amine in measured amount, perhaps in the context of a buffer, the cosmetic formulation can be tweaked to release a finite amount of amine fragrance on rub-in, yet avoid any lingering or continuous odor release.

The second amine is preferred to have a molecular weight of at least 100, preferably greater than 120, more preferably at least 149 and optimally at least 180.

Representative of the second amine are polyethyleneimine, triethanolamine, tris(hydroxymethyl)aminomethane, triisopropanolamine, triethyleneglycol tetramine, tyrosine, glutamine, lysine, arginine and combinations thereof.

Advantageously but not necessarily the second amine will have a pKa no larger than the pKa of the first amine in released form. Typical pKa values are 7.82 and 8.30 respectively for such preferred second amines as triethanolamine and tris(hydroxymethyl)aminomethane (alternatively known as tromethamine). Typical of the first amine is dimethylaminoethanol with pKa of 9.81.

First amines of the present invention are ammonia and dimethylethanolamine. Typical salts of the first amine include ammonium malonate, diammonium malonate, dimethylethanolammonium malonate and bis(dimethylethanolammonium)malonate.

Malonate salts of this invention can either be the half or fully neutralized salts or combinations thereof as represented by general formulas (I) and (II):

[HO₂C (CH₂) CO₂]⁻ [X]⁺ I

⁺[X]₂[O₂C(CH₂) CO₂]⁻² II

wherein X is a protonated first amine.

The present invention can utilize as the first amine neutralized salt, the mono-salt I, di-salt II or mixtures of these salts. When mixtures are present the molar ratio of mono-salt I to di-salt II may range from 1000:1 to 1:1000, preferably from 10:1 to 1:500, more preferably from 2:1, to 1:200, optimally from 1:1 to 1:20.

Amounts of the first amine neutralized malonate salt may range from 0.0001% to 30%, preferably from 0.1% to 15%, more preferably from 0.5% to 10%, optimally from 1% to 8% by weight of the cosmetic composition.

Compositions of this invention will also include a cosmetically acceptable carrier. Amounts of the carrier may range from 1% to 99.9%, preferably from 70% to 95%, optimally from 80% to 90%. Among the useful carriers are water, emollients, fatty acids, fatty alcohols, humectants, thickeners and combinations thereof. The carrier may be aqueous, anhydrous or an emulsion. Preferably the compositions are aqueous, especially water and oil emulsions of the W/O or O/W variety. Water when present may be in amounts ranging from 5% to 95%, preferably from 20% to 70%, optimally from 35% to 60% by weight.

Emollient materials may serve as cosmetically acceptable carriers. These may be in the form of silicone oils, synthetic esters and hydrocarbons. Amounts of the emollients may range anywhere from 0.1% to 95%, preferably between 1% and 50% by weight.

Silicone oils may be divided into the volatile and nonvolatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from 5 x 10⁻⁵ to 0.1 m²/s at 25°C. Among the preferred non-volatile emollients useful in the present compositions are the polydimethyl siloxanes having viscosities from 1 x 10⁻⁵ to 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the suitable ester emollients are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(4) Wax esters such as beeswax, spermaceti wax and tribehenin wax.
(5) Sterols esters, of which cholesterol fatty acid esters are examples thereof.
(6) Sugar ester of fatty acids such as sucrose polybehenate and sucrose polycottonseedate.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5% to 50%, preferably between 1% and 15% by weight of the composition.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums.

Inorganics may also be utilized as thickeners, particularly clays such as bentonites and hectorites, fumed silicas, and silicates such as magnesium aluminum silicate (Veegum®).

Amounts of the thickener may range from 0.0001% to 10%, usually from 0.001% to 1%, optimally from 0.01% to 0.5% by weight.

Cosmetic compositions of the present invention may be in any form. These forms may include lotions, creams, roll-on formulations, sticks, mousses, aerosol and non-aerosol sprays and fabric (e.g. nonwoven textile)-applied formulations.

Surfactants may also be present in cosmetic compositions of the present invention. Total concentration of the surfactant when present may range from 0.1% to 40%, preferably from 1% to 20%, optimally from 1% to 5% by weight of the composition. The surfactant may be selected from the group consisting of anionic, nonionic, cationic and amphoteric actives.

Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionate, C₈-C₂₀ alkyl ether phosphates, C₈-C₂₀ sarcosinates and combinations thereof.

Sunscreen actives may also be included in compositions of the present invention. Particularly preferred are such materials as ethylhexyl p-methoxycinnamate, available as Parsol MCX®, Avobenzene, available as Parsol 1789® and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.1% to 30%, preferably from 2% to 20%, optimally from 4% to 10% by weight.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability.

Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may include vitamins. Illustrative vitamins are Vitamin A (retinol), Vitamin B₂, Vitamin B₆, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001% to 10%, preferably from 0.01% to 1%, optimally from 0.1% to 0.5% by weight.

Another type of useful substance can be that of an enzyme such as oxidases, proteases, lipases and combinations. Particularly preferred is superoxide dismutase, commercially available as Biocell SOD from the Brooks Company, USA.

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from 0.1% to 10%, preferably from 0.5% to 2% by weight of the compositions.

A variety of herbal extracts may optionally be included in compositions of this invention. Illustrative are green tea, chamomile, licorice and extract combinations thereof. The extracts may either be water soluble or water-insoluble carried in a solvent which respectively is hydrophilic or hydrophobic. Water and ethanol are the preferred extract solvents.

Also included may be such materials as retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be utilized but for many compositions of the present invention may also be excluded. Amounts of these materials may range from 0.000001% to 10%, preferably from 0.0001% to 1% by weight.

Colorants, fragrances, opacifiers and abrasives may also be included in compositions of the present invention. Each of these substances may range from 0.05% to 5%, preferably between 0.1% and 3% by weight.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

### EXAMPLES

The following examples will more fully illustrate the embodiments of this invention. All parts, percentages and proportions referred to herein and in the appended claims are by weight unless otherwise illustrated.

### Example 1

A typical cosmetic cream according to the present invention is outlined under Table I.

**TABLE I**

| INGREDIENT | WEIGHT % |
|---|---|
| PHASE A | |
| Water | Balance |
| Disodium EDTA | 0.05 |
| Methyl Paraben | 0.15 |
| Magnesium Aluminum Silicate | 0.60 |
| Triethanolamine | 1.20 |

| PHASE B | |
|---|---|
| Xanthan Gum | 0.20 |
| Natrosol® 250HHR (ethyl cellulose) | 0.50 |
| Butylene Glycol | 3.00 |
| Glycerin | 2.00 |

| PHASE C | |
|---|---|
| Sodium Stearoyl Lactylate | 0.10 |
| Glycerol Monostearate | 1.50 |
| Stearyl Alcohol | 1.50 |
| Isostearyl Palmitate | 3.00 |
| Silicone Fluid | 1.00 |
| Cholesterol | 0.25 |
| Sorbitan Stearate | 1.00 |
| Butylated Hydroxy Toluene | 0.05 |
| Vitamin E Acetate | 0.01 |
| PEG-100 Stearate | 2.00 |
| Stearic Acid | 3.00 |
| Propyl Paraben | 0.10 |
| Parsol MCX® | 2.00 |
| Caprylic/Capric Triglyceride | 0.50 |
| Hydroxycaprylic Acid | 0.01 |
| C12-15 Alkyl Octanoate | 3.00 |

| PHASE D | |
|---|---|
| Diammonium Malonate | 3.00 |

| PHASE E | |
|---|---|
| Vitamin A Palmitate | 0.10 |
| Bisabolol | 0.01 |
| Vitamin A Acetate | 0.01 |
| Fragrance | 0.03 |
| Retinol 50C | 0.02 |

### Example 2

A water-in-oil topical liquid make-up foundation according to the present invention is described in Table II below.

**TABLE II**

| INGREDIENT | WEIGHT % |
|---|---|
| PHASE A | |
| Cyclomethicone | 9.25 |
| Cetyl Octanoate | 2.00 |
| Dimethicone Copolyol | 20.00 |

| PHASE B | |
|---|---|
| Talc | 3.38 |
| Pigment (Iron Oxides) | 10.51 |
| Spheron L-1500 (Silica) | 0.50 |

| PHASE C | |
|---|---|
| Synthetic Wax Durachem 0602 | 0.10 |
| Arachidyl Behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl Paraben | 0.25 |

| PHASE F | |
|---|---|
| Fragrance | 0.05 |

| PHASE G | |
|---|---|
| Water | Balance |
| Ammonium Malonate | 3.00 |
| Tyrosine | 0.50 |
| Methyl Paraben | 0.12 |
| Propylene Glycol | 8.00 |
| Niacinamide | 4.00 |
| Glycerin | 3.00 |
| Sodium Chloride | 2.00 |
| Sodium Dehydroacetate | 0.30 |

### Example 3

Illustrated herein is a skin cream according to the present invention.

**TABLE III**

| INGREDIENT | WEIGHT % |
|---|---|
| Glycerin | 6.93 |
| Niacinamide | 5.00 |
| Dimethylethanolammonium Malonate | 5.00 |
| Tris(hydroxymethyl) Amino Methane | 1.00 |
| Permethyl 101A¹ | 3.00 |
| Sepigel 305² | 2.50 |
| Q2-1403³ | 2.00 |
| Isopropyl Isostearate | 1.33 |
| Arlatone 2121⁴ | 1.00 |
| Cetyl Alcohol CO-1695 | 0.72 |
| SEFA Cottonate⁵ | 0.67 |
| Tocopherol Acetate | 0.50 |
| Panthenol | 0.50 |
| Stearyl Alcohol | 0.48 |
| Titanium Dioxide | 0.40 |
| Disodium EDTA | 0.10 |
| Glydant Plus⁶ | 0.10 |
| PEG-100 Stearate | 0.10 |
| Stearic Acid | 0.10 |
| Purified Water | Balance |

| | |
|---|---|
| Isohexadecane, Presperse Inc., South Plainfield, NJ | |
| ² Polyacrylamide(and)C13-14 Isoparaffin(and) Laureth-7, Seppic Corporation, Fairfield, NJ | |
| ³ dimethicone(and)dimethiconol, Dow Corning Corp. Midland, MI | |
| ⁴ Sorbitan Monostearate and Sucrococoate, ICI Americas Inc., Wilmington, DE | |
| 5 Sucrose ester of fatty acid | |
| ⁶ DMDM Hydantoin (and) Iodopropynyl Butylcarbamate, Lonza Inc., Fairlawn, NJ | |

### Example 4

A relatively anhydrous composition according to the present invention is reported in Table V.

**TABLE IV**

| INGREDIENT | WEIGHT % |
|---|---|
| Cyclomethicone | 80.45 |
| Dimethicone | 9.60 |
| Squalane | 6.00 |
| Isostearic Acid | 1.90 |
| Borage Seed Oil | 0.90 |
| Ammonium Malonate (50% in water) | 0.50 |
| Triethyleneglycol Tetramine | 0.20 |
| Retinyl Palmitate | 0.25 |
| Ceramide 6 | 0.10 |
| Tocopherol | 0.10 |

### Example 5

An aerosol packaged foaming cleanser suitable for the present invention is outlined in Table VI.

**TABLE V**

| INGREDIENT | WEIGHT % |
|---|---|
| Sunflower Seed Oil | 20.00 |
| Maleated Soybean Oil | 5.00 |
| Silicone Urethane | 1.00 |
| Polyglycero-4 Oleate | 1.00 |
| Sodium C14-16 Olefin Sulfonate | 15.00 |
| Sodium Lauryl Ether Sulphate (25% active) | 15.00 |
| Cocoamidopropylbetaine | 15.00 |
| DC 1784® (Silicone Emulsion 50%) | 5.00 |
| Polyquaternium-11 | 1.00 |
| Bis(dimethylethanolammonium) Malonate | 1.00 |
| Triethanolamine | 0.20 |
| Water | Balance |

An aerosol is prepared using 92% by weight of the concentrate in Table VI and 8% propellant, the latter being a combination of dimethylether, isobutane and propane.

### Example 6

An adhesive cosmetic patch may also be formulated according to the present invention. An adhesive hydrogel is prepared by mixing 30 grams of 2-acrylamido-2-methylpropane sulphonic acid monomer in 20 grams distilled water and 5 grams of a 1% aqueous solution of methylene-bis-acrylamide. The solution is then activated with 0.4% magnesium persulphate catalyst. Shortly after mixing the catalyst with the hydrogel solution, 0.1 grams ammonium malonate and 0.02 grams tris(hydroxymethyl)amino methane in 5 ml water is added. The resultant solution is coated onto a 50/50 blend of polypropylene and hydrophilic polyester and allowed to solidify. The resulting deposited hydrogel is warmed for 24 hours at 40°C in a hot air oven. Final water content of the hydrogel is 50%. A polystyrene backing layer is laid over the adhesive hydrogel.

### Example 7

A disposable, single use personal towelette product is described according to the present invention. A 70/30 polyester/rayon non-woven towelette is prepared with a weight of 1.8 grams and dimensions of 15 cm by 20 cm. Onto this towelette is impregnated a composition as outlined in Table VII below.

**TABLE VI**

| INGREDIENT | WEIGHT % |
|---|---|
| Ammonium Malonate | 5.50 |
| Tyrosine | 2.00 |
| Glycerin | 2.00 |
| Hexylene Glycol | 2.00 |
| Disodium Capryl Amphodiacetate | 1.00 |
| Gluconolactone | 0.90 |
| Silicone Microemulsion | 0.85 |
| Witch Hazel | 0.50 |
| PEG-40 Hydrogenated Castor Oil | 0.50 |
| Fragrance | 0.20 |
| Vitamin E Acetate | 0.001 |
| Water | Balance |

The foregoing description and examples illustrate selected embodiments of the present invention.

## Claims

1. A cosmetic composition comprising:
(i) a malonate salt formed from a first amine which is ammonia or dimethylaminoethanol and malonic acid;
(ii) a second amine different from the first amine and having a molecular weight no lower than 100;
(iii) from 1 to 99.9% by weight of a cosmetically acceptable carrier.

2. The composition according to claim 1 wherein the second amine is selected from polyethyleneimine, triethanolamine, tris(hydroxymethyl) amino methane, triisopropanolamine, triethylene glycol tetramine, tyrosine, glutamine, lysine, arginine and combinations thereof.

3. The composition according to any of the preceding claims wherein the second amine is an amino acid.

4. The composition according to any of the preceding claims wherein the second amine has a pKa no larger than a pKa of the first amine.

5. The composition according to any of the preceding claims wherein the second amine has a molecular weight of at least 149.

6. The composition according to any of the preceding claims wherein the first amine is dimethylaminoethanol.

7. The composition according to any of the preceding claims wherein the first amine is ammonia.

8. A cosmetic patch or towelette product containing a cosmetic composition according to any of claims 1 to 7.

## Patentansprüche

1. Kosmetische Zusammensetzung, umfassend:
(i) ein Malonatsalz, gebildet aus einem ersten Amin, das Ammoniak oder Dimethylaminoethanol darstellt und Malonsäure;
(ii) ein zweites Amin, das von dem ersten Amin verschieden ist und ein Molekulargewicht nicht niedriger als 100 aufweist;
(iii) 1 bis 99,9 Gew.-% eines kosmetisch verträglichen Trägers.

2. Zusammensetzung nach Anspruch 1, worin das zweite Amin aus Polyethylenimin, Triethanolamin, Tris(hydroxymethyl)amino-methan, Triisopropanolamin, Triethylenglykoltetramin, Tyrosin, Glutamin, Lysin, Arginin und Kombinationen davon ausgewählt ist.

3. Zusammensetzung nach einem der vorangehenden Ansprüche, worin das zweite Amin eine Aminosäure darstellt.

4. Zusammensetzung nach einem der vorangehenden Ansprüche, worin das zweite Amin einen pKa-Wert nicht größer als einen pKa-Wert von dem ersten Amin aufweist.

5. Zusammensetzung nach einem der vorangehenden Ansprüche, worin das zweite Amin ein Molekulargewicht von mindestens 149 aufweist.

6. Zusammensetzung nach einem der vorangehenden Ansprüche, worin das erste Amin Dimethylaminoethanol darstellt.

7. Zusammensetzung nach einem der vorangehenden Ansprüche, worin das erste Amin Ammoniak darstellt.

8. Kosmetikpflaster- oder Feuchtigkeitstuchprodukt, enthaltend eine kosmetische Zusammensetzung nach einem der Ansprüche 1 bis 7.

## Revendications

1. Composition cosmétique comprenant :
(i) un sel de malonate formé à partir d'une première amine qui est de l'ammoniac ou du diméthyl aminoéthanol et de l'acide malonique ;
(ii) une seconde amine différente de la première amine et ayant une masse moléculaire qui n'est pas inférieure à 100 ;
(iii) de 1 à 99,9 % en poids d'un matériau formant support acceptable d'un point de vue cosmétique.

2. Composition selon la revendication 1, dans laquelle la seconde amine est sélectionnée à partir du polyéthylène imine, de la triéthanolamine, du tris (hydroxyméthyl) amino méthane, de la triisopropanolamine, de la triéthylène glycol tétramine, de la tyrosine, de la glutamine, de la lysine, de l'arginine et des combinaisons de ceux-là.

3. Composition selon l'une quelconque des revendications précédentes, dans laquelle la seconde amine est un acide aminé.

4. Composition selon l'une quelconque des revendications précédentes, dans laquelle la seconde amine a une valeur pKa qui n'est pas plus grande qu'une valeur pKa de la première amine.

5. Composition selon l'une quelconque des revendications précédentes, dans laquelle la seconde amine a une masse moléculaire d'au moins 149.

6. Composition selon l'une quelconque des revendications précédentes, dans laquelle la première amine est du diméthyl aminoéthanol.

7. Composition selon l'une quelconque des revendications précédentes dans laquelle la première amine est de l'ammoniac.

8. Produit de timbre ou de lingette cosmétique contenant une composition cosmétique selon l'une quelconque des revendications 1 à 7.
